# FASCICULE DE BREVET EUROPEEN

(11) **EP 0 756 862 B1**
(45) Date de publication et mention de la délivrance du brevet: **15.04.1998**
(21) Numéro de dépôt: 96401277.7
(22) Date de dépôt: 12.06.1996
(51) Int. Cl.: A61K 7/48

(54) **Utilisation d'un antagoniste de bradykinine dans une composition cosmétique, pharmaceutique ou dermatologique et composition obtenue**
Verwendung eines Bradykinin-Antagonisten in einer kosmetischen, pharmazeutischen oder dermatologischen Zusammensetzung und die erhaltene Zusammensetzung
Use of bradykinine antagonist in a cosmetic, pharmaceutical or dermatological composition and the composition obtained

(30) Priorité: 31.07.1995 FR 9509304
(43) Date de publication de la demande: 05.02.1997
(73) Titulaire: L'OREAL, 75008 Paris (FR)
(72) Inventeur: Breton, Lionel, 78000 Versailles (FR); de Lacharriere, Olivier, 75015 Paris (FR)
(74) Mandataire: Lhoste, Catherine

(56) Documents cités:
- EP-A- 0 552 106
- EP-A- 0 661 058
- WO-A-83/01252
- WO-A-93/14084
- GB-A- 2 271 774
- NEUROSCIENCE, vol. 48, no. 4, 1992, pages 963-968, XP002000489 T.L. BUCKLEY ET AL:
- BRITISH JOURNAL OF PHARMACOLOGY, vol. 110, no. 2, Octobre 1993, pages 772-776, XP002000490 K.J. ESCOTT ET AL:
- BRITISH JOURNAL OF PHARMACOLOGY, vol. 104, no. 3, Novembre 1991, pages 738-742, XP002000491
- BRITISH JOURNALOF PHARMACOLOGY, vol. 109, no. 1, 1993, pages 259-264, XP002000492 S.M. MOUSSAOUI:
- BRITISH JOURNAL OF DERMATOLOGY, vol. 124, no. 4, 1991, pages 324-328, XP002000493 J. WALLENGREN:
- CONTACT DERMATITIS, vol. 19, no. 5, 1988, pages 351-354, XP002000494 JOANNA WALLENBERG ET AL:

## Description

La présente invention se rapporte à l'utilisation d'un antagoniste de bradykinine dans une composition cosmétique, pharmaceutique ou dermatologique à application topique, destinée notamment au traitement des peaux sensibles, ainsi qu'à une composition contenant un antagoniste de bradykinine en vue de diminuer voire éliminer les effets irritants de certains produits et notamment de certains actifs utilisés dans le domaine cosmétique, pharmaceutique ou dermatologique.

Il est connu que certaines peaux sont plus sensibles que d'autres. Les symptômes des peaux sensibles étaient jusqu'à présent mal caractérisés et le problème de ces peaux était, de ce fait, mal défini ; personne ne connaissait exactement le processus mis en cause dans la sensibilité -hyperréactivité cutanée non allergique- de la peau. Certains pensaient qu'une peau sensible était une peau qui réagissait aux produits cosmétiques, d'autres qu'il s'agissait d'une peau qui réagissait à plusieurs facteurs extérieurs, pas forcément liés aux produits cosmétiques et/ou dermatologiques.

Certains tests ont été essayés pour tenter de cerner les peaux sensibles, par exemple des tests à l'acide lactique et au DMSO qui sont connus pour être des substances irritantes : voir par exemple l'article de K. Lammintausta et al., Dermatoses, 1988, 36, pages 45-49; et l'article de T. Agner et J. Serup, Clinical and Experimental Dermatology, 1989, 14, pages 214-217. Mais ces tests ne permettaient pas de caractériser complètement les peaux sensibles.

Par ailleurs, on assimilait les peaux sensibles à des peaux allergiques.

Du fait que l'on connaissait mal les caractéristiques des peaux sensibles, il était jusqu'à présent très difficile de les traiter, et on les traitait indirectement, par exemple en limitant dans les compositions cosmétiques ou dermatologiques l'emploi de produits à caractère irritant tels que les tensioactifs, les conservateurs ou les parfums ainsi que certains actifs.

La demanderesse a réalisé de nombreux tests cliniques et a su déterminer les symptômes liés aux peaux sensibles. Ces symptômes sont en particulier des signes subjectifs, qui sont essentiellement des sensations dysesthésiques. On entend par sensations dysesthésiques des sensations plus ou moins douloureuses ressenties dans une zone cutanée comme les picotements, fourmillements, démangeaisons ou prurits, brûlures, échauffements, inconforts, tiraillements, etc.

La demanderesse a pu montrer en outre qu'une peau sensible n'était pas une peau allergique. En effet, une peau allergique est une peau qui réagit à un agent extérieur, un allergène, qui déclenche une réaction d'allergie. Il s'agit d'un processus immunologique qui ne se produit que lorsqu'un allergène est présent et qui ne touche que les sujets sensibilisés. La caractéristique essentielle de la peau sensible est selon la demanderesse, au contraire, un mécanisme de réponse à des facteurs extérieurs, qui peut concerner tout individu, même si les individus dits à peau sensible y réagissent plus vite que les autres. Ce mécanisme n'est pas immunologique.

La demanderesse a maintenant trouvé que les peaux sensibles pouvaient être scindées en deux grandes formes cliniques, les peaux irritables et les peaux intolérantes.

Une peau irritable est une peau qui réagit par un prurit, c'est-à-dire par des démangeaisons ou par des picotements à différents facteurs tels que l'environnement, les émotions, les aliments, le vent, les frottements, le rasoir, le savon, les tensioactifs, l'eau dure à forte concentration de calcaire, les variations de température ou la laine. En général, ces signes sont associés à une peau sèche avec ou sans dartres, ou à une peau qui présente un érythème.

Une peau intolérante est une peau qui réagit par des sensations d'échauffement, de tiraillements, mais également au même titre que les peaux irritables par un prurit, c'est-à-dire par des démangeaisons ou des picotements. La peau intolérante se caractérise également par des fourmillements et/ou des rougeurs, à différents facteurs tels que l'environnement, les émotions, les aliments. En général, ces signes sont associés à un érythème et à une peau avec ou sans dartres.

D'une manière générale, les peaux sensibles se définissent par une réactivité particulière de la peau. Cette hyper-réactivité peut être notamment mise en jeu par des facteurs environnementaux, émotionnels, alimentaires ou encore par l'application ou le contact de produits cosmétiques ou dermatologiques. Cet état d'hyper-réactivité qui définit les peaux sensibles différencie celles-ci de la réactivité ubiquitaire provoquée par des agents irritants qui induisent une irritation de la peau chez la quasitotalité des individus.

Cet état d'hyper-réactivité est ressentie et reconnue par les individus qui en sont atteints comme une 〈〈 peau sensible 〉〉.

Les cuirs chevelus "sensibles" ont une sémiologie clinique plus univoque : les sensations de prurit et/ou de picotements et/ou d'échauffements sont essentiellement déclenchés par des facteurs locaux tels que frottements, savon, tensioactifs, eau dure à forte concentration de calcaire, shampooings ou lotions. Ces sensations sont aussi parfois déclenchées par des facteurs tels que l'environnement, les émotions et/ou les aliments. Un érythème et une hyperséborrhée du cuir chevelu ainsi qu'un état pelliculaire sont fréquemment associés aux signes précédents.

Par ailleurs, dans certaines régions anatomiques comme les grands plis (régions inguinales, génitale, axillaires, poplitées, anale, sous-mammaires, plis du coude) et les pieds, la peau sensible se traduit par des sensations prurigineuses et/ou des sensations dysesthésiques (échauffement, picotements) liées en particulier à la sueur, aux frottements, à la laine, aux tensioactifs, à l'eau dure à forte concentration en calcaire et/ou aux variations de température.

Pour déterminer si une peau est sensible ou non, la demanderesse a également mis au point un test. En effet, après avoir effectué un grand nombre de tests dans le but de définir une peau sensible, elle a trouvé de manière surprenante qu'il existait un lien entre les personnes à peau sensible et celles qui réagissaient à une application topique de capsaïcine.

Le test à la capsaïcine consiste à appliquer sur environ 4 cm² de peau 0,05 ml d'une crème contenant 0,075 % de capsaïcine et à noter l'apparition de signes subjectifs provoqués par cette application, tels que picotements, brûlures et démangeaisons. Chez les sujets à peaux sensibles, ces signes apparaissent entre 3 et 20 minutes après l'application et sont suivis de l'apparition d'un érythème qui débute à la périphérie de la zone d'application.

La capsaïcine était utilisée comme médicament, en particulier pour traiter les douleurs du zona. La capsaïcine provoque un relargage des neuropeptides à partir des fibres nerveuses sensitives, et en particulier des tachykinines et du CGRP (peptide dérivé du gène de la calcitonine: Calcitonin Gene Related Peptide en terminologie anglosaxonne) qui proviennent de terminaisons nerveuses de l'épiderme et du derme. La demanderesse a constaté que le schéma physiopathologique commun à tous les états des peaux sensibles était lié à une grande aptitude à libérer des neuropeptides et plus particulièrement de la substance P et du CGRP dans la peau. On sait, en outre, que ces neuropeptides libérés par les terminaisons sensitives épidermiques induit une cascade d'événements biochimiques dont les premiéres étapes concernent les mastocytes. La fixation de ces neuropeptides et notamment la substance P sur les récepteurs mastocytaires induit une libération de nombreux médiateurs pro-inflammatoires.

La demanderesse a maintenant découvert que les caractéristiques essentielles des peaux sensibles (réactions d'irritation et d'intolérance cutanée) étaient liées notamment à la libération de ces neuropeptides, induite par la bradykinine en se fixant notamment sur les fibres nerveuses contenant de la substance P et du CGRP.

La bradykinine est un nonapeptide inflammatoire d'origine plasmatique libéré à partir d'un précurseur kininogène par une protéase plasmatique du nom de Kallikreine (EC 3.4.21.24). La bradykinine est impliquée dans un grand nombre de désordres physiopathologiques parmi lesquels : hypotension, contraction des muscles lisses des tractus digestif et respiratoire et de l'utérus, la douleur, la prolifération du tissu conjonctif et la libération de différents médiateurs de l'inflammation : cytokines, leukotriènes et prostaglandines.

L'action de la bradykinine s'exerce par sa fixation sur deux types de récepteurs appelés récepteurs B₁ et B₂ à la bradykinine.

Par ailleurs, la libération de bradykinine peut provoquer directement et indépendamment des processus décrit ci-dessous, une réaction inflammatoire qui se traduit par un érythème, un oedéme et un prurit.

Aussi, la demanderesse a constaté que l'utilisation d'antagoniste de bradykinine pouvait être utilisée dans le traitement préventif et/ou curatif des peaux sensibles éventuellement en inhibant la libération de substance P et/ou de CGRP, eux mêmes responsables de la libération de différents médiateurs de l'inflammation responsables de la réactivité des peaux sensibles.

Pour traiter les peaux sensibles, la demanderesse a donc envisagé d'utiliser des. antagonistes de bradykinine. Elle a en effet constaté de manière surprenante que l'incorporation d'antagonistes de bradykinine dans une composition cosmétique, pharmaceutique ou dermatologique permet d'éviter l'irritation et/ou les sensations dysesthésiques et/ou les prurits de la peau et/ou des muqueuses.

La présente invention a donc pour objet l'utilisation d'au moins un composé choisi parmi les antagonistes de bradykinine, dans une composition à application topique, contenant un milieu cosmétiquement, pharmaceutiquement ou dermatologiquement acceptable, pour traiter les peaux sensibles.

La présente invention a encore pour objet l'utilisation dans une composition topique d'au moins composé choisi parmi les antagonistes de bradykinine pour prévenir et/ou lutter contre les irritations cutanées et/ou les dartres et/ou les érythèmes et/ou les sensations d'échauffement et/ou de dysesthésie et/ou les prurits de la peau et/ou des muqueuses.

On entend par 〈〈 antagoniste 〉〉 de bradykinine toute substance susceptible d'inhiber la libération et/ou la synthèse et/ou la fixation réceptorielle de bradykinine. Les antagonistes inhibant la fixation réceptorielle de bradykinine sont des agents spécifiques du récepteur de type 1 (B₁) et/ou du type 2 (B₂) de la bradykinine.

Selon l'invention, il est possible d'utiliser un seul ou conjointement plusieurs antagonistes de bradykinine. Par exemple, il est possible d'utiliser un antagoniste de libération et/ou de synthèse en association avec un antagoniste réceptoriel B₁ et/ou B₂, par exemple.

En outre, la demanderesse a constaté que l'adjonction d'antagonistes de bradykinine dans des compositions cosmétiques, pharmaceutiques ou dermatologiques, à application topique, contenant des produits irritants (alphahydroxy-acides, rétinoïdes, peroxyde de benzoyle,...) permettait également de diminuer, voire de supprimer les réactions d'irritations habituellement provoquées par ces produits. Ces réactions d'irritations se traduisent dans les instants qui suivent l'application par des sensations dysesthésiques (échauffement, sensations de brûlures, démangeaisons ou prurit, sensations de picotements, de tiraillements...), et/ou par des rougeurs, et/ou par de l'oedème. Ces états d'irritation peuvent également se traduire à distance de l'application par la persistance, l'apparition ou la réapparition des sensations dysesthésiques suscitées et/ou par des rougeurs et/ou des squames ; ces états d'irritation de la peau peuvent prendre l'aspect de plaques de xérose cutanée et/ou de dartres.

Aussi, l'adjonction d'antagonistes de bradykinine, dans des compositions cosmétiques, pharmaceutiques ou dermatologiques irritantes permet également de diminuer, voire de supprimer les réactions d'irritations habituellement provoquées par certains produits.

L'invention a donc encore pour objet une composition contenant dans un milieu cosmétiquement, pharmaceutiquement ou dermatologiquement acceptable au moins un produit à effet secondaire irritant, caractérisée en ce qu'elle contient au moins un agent antagoniste de cet effet choisi parmi les antagonistes de bradykinine.

Un milieu cosmétiquement, dermatologiquement ou pharmaceutiquement acceptable est un milieu qui est compatible avec la peau, le cuir chevelu, les ongles, les muqueuses. La composition contenant un antagoniste de bradykinine peut donc être appliquée sur le visage, le cou, les cheveux et les ongles, ou toute autre zone cutanée du corps comme les grands plis (régions axillaires, sous-mammaires, plis du coude etc.).

Pour qu'une substance soit reconnue comme un antagoniste réceptoriel de bradykinine, elle doit répondre notamment, aux caractéristiques suivantes :
- avoir une affinité sélective pour les récepteurs spécifiques de ce composé : de type B₁ et/ou B₂.
   Les modèles expérimentaux utilisés sont réalisés sur culture de cellules de l'aorte mésentérique (fixation réceptorielle sur récepteurs B₁ selon la méthode décrite par J.P. Galizzi publiée dans Brit. J. Pharmacol, 1994, 113, 389) et/ou sur intestin (fixation réceptorielle sur récepteurs B₂.selon la méthode décrite par Burch R.M. publiée dans Biotech. Update (Dupont-Nen),1992, 7, 2.
- avoir une activité pharmacologique antagoniste réceptoriel de bradykinine, c'est-à-dire induire une réponse pharmacologique cohérente dans des tests spécifiques.
   L'activité pharmacologique est dans ce cas, évaluée sur organes isolés selon la méthode décrite par Rhaleb N.E. et al. dans Brit. J. Pharmacol., 1990, 94, 445 en ce qui concerne une activité antagoniste de type B₁ et/ou B₂.

Pour qu'une substance soit reconnue comme un antagoniste de la libération et/ou de la synthèse de bradykinine, elle doit répondre notamment à la caractéristique suivante :
- inhibition de la libération de bradykinine

Les antagonistes de bradykinine utilisables dans l'invention sont notamment, ceux classiquement utilisés dans le traitement des états allergiques, des états inflammatoires, des brulûres, des chocs septiques.

Les composés peuvent être par exemple, l'Icatibant, l'HOE140, le CP0364, le CP0127, le NPC-17731, ainsi que les composés cités dans les documents EP578521, US5212182, EP564972, EP548825, JP93255107, EP552106, FR2686343, WO9311789.

Dans les compositions selon l'invention, les antagonistes de bradykinine sont utilisés de préférence en une quantité allant de 0,000001 à 5 % en poids par rapport au poids total de la composition, et en particulier en une quantité allant de 0,0001 à 0,1 % en poids par rapport au poids total de la composition.

Les compositions selon l'invention peuvent se présenter sous toutes les formes galéniques normalement utilisées pour une application topique, notamment sous forme de solutions aqueuses, hydroalcooliques ou huileuses, de dispersions du type lotion ou sérum, de gels anhydres ou lipophiles, d'émulsions de consistance liquide ou semi-liquide du type lait, obtenues par dispersion d'une phase grasse dans une phase aqueuse (H/E) ou inversement (E/H), ou de suspensions ou d'émulsions de consistance molle, semi-solide ou solide du type crème ou gel, ou encore de microémulsions, de microcapsules, de microparticules ou de dispersions vésiculaires de type ionique et/ou non ionique. Ces compositions sont préparées selon les méthodes usuelles.

Elles peuvent être également utilisées pour le cuir chevelu sous forme de solutions aqueuses, alcooliques ou hydroalcooliques, ou sous forme de crèmes, de gels, d'émulsions, de mousses ou encore sous forme de compositions pour aérosol contenant également un agent propulseur sous pression.

Les quantités des différents constituants des compositions selon l'invention sont celles classiquement utilisées dans les domaines considérés.

Ces compositions constituent notamment des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires ou mieux après solaires, des lotions, gels ou mousses pour le soin de la peau, comme des lotions de nettoyage ou de désinfection, des lotions anti-solaires, des lotions de bronzage artificiel, des compositions pour le bain, des compositions déodorantes contenant un agent bactéricide, des gels ou lotions après-rasage, des crèmes épilatoires, des compositions contre les piqûres d'insectes, des compositions anti-douleur ou des compositions pour traiter certaines maladies de la peau comme les prurits sévères, la rosacée, l'acné, les ulcères de la jambe, le psoriasis, les pustules, les vergetures.

Les compositions selon l'invention peuvent également consister en des préparations solides constituant des savons ou des pains de nettoyage.

Les antagonistes de bradykinine peuvent être aussi incorporés dans diverses compositions pour soins ou traitements capillaires, et notamment des shampooings éventuellement antiparasitaires, des lotions de mise en plis, des lotions traitantes, des crèmes ou des gels coiffants, des compositions de teintures (notamment teintures d'oxydation) éventuellement sous forme de shampooings colorants, des lotions restructurantes pour les cheveux, des compositions de permanente (notamment des compositions pour le premier temps d'une permanente), des lotions ou des gels antichute, etc.

Les compositions de l'invention peuvent aussi être à usage bucco-dentaire, par exemple une pâte dentifrice ou un bain de bouche. Dans ce cas, les compositions peuvent contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition de l'invention est une émulsion, la proportion de la phase grasse peut aller de 5 % à 80 % en poids, et de préférence de 5 % à 50 % en poids par rapport au poids total de la composition. Les huiles, les émulsionnants et les coémulsionnants utilisés dans la composition sous forme d'émulsion sont choisis parmi ceux classiquement utilisés dans les domaines cosmétique, pharmaceutique ou dermatologique. L'émulsionnant et le coémulsionnant sont présents, dans la composition, en une proportion allant de 0,3 % à 30 % en poids, et de préférence de 0,5 à 30 % ou mieux de 0,5 à 20 % en poids par rapport au poids total de la composition. L'émulsion peut, en outre, contenir des vésicules lipidiques.

Lorsque la composition de l'invention est une solution ou un gel huileux, la phase grasse peut représenter plus de 90 % du poids total de la composition.

De façon connue, la composition de l'invention peut contenir également des adjuvants habituels dans le domaine cosmétique, pharmaceutique ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes. Les quantités de ces différents adjuvants sont celles classiquement utilisées dans le domaine cosmétique, pharmaceutique ou dermatologique, et par exemple de 0,01 % à 20 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles siliconées (cyclométhicone) et les huiles fluorées (perfluoropolyéthers). On peut aussi utiliser comme matières grasses des alcools gras, des acides gras (acide stéarique), des cires (paraffine, carnauba, cire d'abeilles).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, ou encore l'éthylcellulose, le polyéthylène.

Comme actifs hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon et des extraits bactériens ou végétaux, notamment ceux d'Aloe Vera.

Comme actifs lipophiles, on peut utiliser le rétinol (vitamine A) et ses dérivés, le tocophérol (vitamine E) et ses dérivés, les acides gras essentiels, les céramides, les huiles essentielles.

On peut entre autres associer les antagonistes de bradykinines à des agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, le rétinal, les rétinoïdes, la vitamine D et ses dérivés, les estrogènes tels que l'estradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents anti-inflammatoires stéroïdiens, tels que l'hydrocortisone, les anthralines (dioxyanthranol), les anthranoïdes, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine;
- les agents antiviraux tels que l'acydovir ;
- les agents kératolytiques tels que les acides alpha- et bêta-hydroxy-carboxyliques ou bêta-cétocarboxyliques, leurs sels, amides ou esters et plus particulièrement les alpha-hydroxyacides tels que l'acide glycolique, l'acide lactique, l'acide malique, l'acide tartrique, l'acide citrique et de manière générale les acides de fruits et les bêta-hydroxyacides comme l'acide salicylique et ses dérivés notamment alcoylés comme, l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle;
- les antimétabolites ;
- les agents pour lutter contre la chute des cheveux comme le monoxidil ;
- les antiseptiques.

De façon avantageuse, les antagonistes de bradykinine sont associés à des produits à effet secondaire irritant et notamment des actifs, utilisés couramment dans le domaine cosmétique, pharmaceutique ou dermatologique. La présence d'un antagoniste de bradykinine dans une composition cosmétique, pharmaceutique ou dermatologique contenant un produit voire un actif ayant un effet irritant permet d'atténuer fortement, voire de supprimer cet effet irritant.

En particulier, les antagonistes de bradykinine permettent notamment, d'augmenter la quantité d'actif cosmétique, pharmaceutique ou dermatologique par rapport à la quantité normalement utilisée, en vue d'une efficacité améliorée.

Les produits irritants auxquels s'applique l'invention sont notamment les parfums, les tensioactifs (ioniques ou non-ioniques), les conservateurs, certains filtres solaires, les solvants organiques, les solutions alcooliques et certains actifs cosmétiques, pharmaceutiques ou dermatologiques.

En particulier, les actifs à effet secondaire irritant sont choisis parmi les α-hydroxy-acides (glycolique, lactique, malique, citrique, tartrique, mandélique ), les β-hydroxy-acides (acide salicylique et ses dérivés), les α-céto-acides, les β-céto-acides, les rétinoïdes (rétinol et ses esters, rétinal, acide rétinoïque et ses dérivés, rétinoïdes, notamment ceux décrits dans les documents FR-A-2 570 377, EP-A-199636, EP-A-325540, EP-A-402072), les anthralines (dioxyanthranol), les anthranoïdes, les peroxydes (notamment de benzoyle), le minoxidil, les sels de lithium, les antimétabolites, la vitamine D et ses dérivés, les teintures ou colorants capillaires (paraphényiènediamine et ses dérivés, les aminophénols), les solutions alcooliques parfumantes (parfums, eaux de toilette, après rasage, déodorants), les agents antitranspirants (certains sels d'aluminium), les actifs dépilatoires ou de permanentes (thiols), les dépigmentants (hydroquinone), les actifs antipoux (pyréthrine).

L'emploi d'antagoniste de bradykinine permet notamment, de multiplier de 2 à 10 fois la quantité de produit, et plus spécialement d'actif à effet secondaire irritant par rapport à l'état de la technique, sans ressentir tous les inconforts mentionnés ci-dessus. Ainsi, on peut utiliser les hydroxyacides jusqu'à 50 % du poids de la composition ou les rétinoïdes jusqu'à 5 %, sans aucune gène.

En particulier, la composition contient un antagoniste de bradykinine choisi parmi des molécules organiques minérales ou contenu dans des extraits obtenus à partir de cellule végétale, animale ou à partir de microorganismes.

La présente invention a en outre pour objet un procédé de traitement cosmétique, et/ou dermatologique notamment des peaux sensibles, caractérisé par le fait que l'on applique sur la peau, sur le cuir chevelu, et/ou sur les muqueuses, une composition telle que décrite ci-dessus contenant au moins un antagoniste de bradykinine dans un milieu cosmétiquement et/ou dermatologiquement acceptable.

Le procédé de traitement cosmétique et/ou dermatologique de l'invention peut être mis en oeuvre notamment en appliquant les compositions hygiéniques ou cosmétiques et/ou dermatologiques telles que définies ci-dessus, selon la technique d'utilisation habituelle de ces compositions. Par exemple : application de crèmes, de gels, de sérums, de lotions, de laits de démaquillage ou de compositions après-solaires sur la peau ou sur les cheveux secs, application d'une lotion pour cheveux sur cheveux mouillés, de shampooings ou encore application de dentifrice sur les gencives.

Les exemples suivants illustrent l'invention. Dans ces exemples, les proportions indiquées sont des pourcentages en poids.

### Exemple 1 : lotion démaquillante pour le visage des peaux sensibles

| | |
|---|---|
| Icatibant | 0,005 |
| Antioxydant | 0,05 |
| Isopropanol | 40,00 |
| Conservateur | 0,30 |
| Eau | qsp 100 % |

### Exemple 2: lotion démaquillante pour le visage des peaux sensibles

| | |
|---|---|
| CP0127 | 0,001 |
| Antioxydant | 0,05 |
| Isopropanol | 40,00 |
| Conservateur | 0,30 |
| Eau | qsp 100 % |

### Exemple 3 : Gel pour le soin du visage des peaux sensibles

| | |
|---|---|
| Icatibant | 0,04 |
| Hydroxypropylcellulose (Klucel H vendu par la société Hercules) | 1,00 |
| Antioxydant | 0,05 |
| Isopropanol | 40,00 |
| Conservateur | 0,30 |
| Eau | qsp 100 % |

### Exemple 4 : Crème de soin du visage des peaux sensibles (émulsion huile dans eau)

| | |
|---|---|
| CP0364 | 0,02 |
| Stéarate de glycérol | 2,00 |
| Polysorbate 60 (Tween 60 vendu par la société ICI) | 1,00 |
| Acide stéarique | 1,40 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,5 |
| Conservateur | 0,30 |
| Eau | qsp 100 % |

### Exemple 5 : Crème de soin antirides pour le visage des peaux sensibles (émulsion huile dans eau)

| | |
|---|---|
| CP0364 | 0,15 |
| Stéarate de glycérol | 2,00 |
| Polysorbate 60 (Tween 60 vendu par la société ICI) | 1,00 |
| Acide stéarique | 1,40 |
| Acide n-octanoyl-5-salicylique | 0,50 |
| Triéthanolamine | 0,70 |
| Carbomer | 0,40 |
| Fraction liquide du beurre de karité | 12,00 |
| Perhydrosqualène | 12,00 |
| Antioxydant | 0,05 |
| Parfum | 0,50 |
| Conservateur | 0,30 |
| Eau | qsp 100 % |

### Exemple 6 : Gel émulsionné de soin contre les piqûres d'insectes (émulsion huile dans eau)

| | |
|---|---|
| Icatibant | 3,00 |
| Huile de Purcellin (vendue par la Société Dragocco) | 7,00 |
| PEG-6/PEG-32/Glycol Stéarate (Tefose^{R} 63 de Gattefosse) | 0,30 |
| Conservateur | 0,30 |
| Parfum | 0,40 |
| Carbomer | 0,60 |
| Crotamiton | 5,00 |
| Acide glycyrrhétinique | 2,00 |
| Alcool éthylique | 5,00 |
| Triéthanolamine | 0,20 |
| Eau | qsp 100 % |

### Exemple 7 : Gel anti-douleur

| | |
|---|---|
| CP0364 | 0,03 |
| Hydroxypropylcellulose (Klucel H vendu par la société Hercules) | 1,00 |
| Antioxydant | 0,05 |
| Chlorhydrate de lidocaïne | 2,00 |
| Isopropanol | 40,00 |
| Conservateur | 0,30 |
| Eau | qsp 100 % |

### Exemple 8 : Crème de soin de l'érythème solaire des peaux sensibles (émulsion huile dans eau)

| | |
|---|---|
| CP0127 | 0,25 |
| Stéarate de glycérol | 2,00 |
| Polysorbate 60 (Tween 60 vendu par la société ICI) | 1,00 |
| Acide stéarique | 1,40 |
| Acide glycyrrhétinique | 2,00 |
| Triéthanolamine | 0,70 |
| Carbomer | 0,40 |
| Fraction liquide du beurre de karité | 12,00 |
| Huile de tournesol | 10,00 |
| Antioxydant | 0,05 |
| Parfum | 0,5 |
| Conservateur | 0,30 |
| Eau | qsp 100 % |

### Exemple 9 : Crème de soin antirides pour le visage des peaux sensibles (émulsion huile dans eau)

Cet exemple se différencie de l'exemple 5, par le remplacement de l'acide n-octanoyl-5-salicylique par un mélange d'acide de fruit (acides lactique, glycolique, tartrique, citrique, malique).

### Exemple 10 : Gel pour traiter l'acné

| | |
|---|---|
| Acide all trans rétinoïque | 0,05 |
| Icatibant | 0,55 |
| Hydroxypropylcellulose (Klucel H vendu par la société Hercules) | 1,00 |
| Antioxydant | 0,05 |
| Isopropanol | 40,00 |
| Conservateur | 0,3 |
| Eau | qsp 100 % |

## Revendications

1. Utilisation d'au moins un composé choisi parmi les antagonistes de bradykinine, dans une composition contenant un milieu cosmétiquement, dermatologiquement ou pharmaceutiquement acceptable pour traiter les peaux sensibles.

2. Utilisation selon la revendication 1, caractérisée en ce que le composé est utilisé en une quantité allant de 0,000001 à 5 % en poids par rapport au poids total de la composition.

3. Utilisation selon l'une quelconque des revendications précédentes, caractérisée en ce que le composé est utilisé en une quantité allant de 0,0001 à 0,1 % en poids par rapport au poids total de la composition.

4. Utilisation selon l'une quelconque des revendications précédentes, caractérisée en ce que le milieu cosmétiquement, pharmaceutiquement ou dermatologiquement acceptable est une solution aqueuse, huileuse ou hydroalcoolique, une émulsion eau-dans-huile, une émulsion huile-dans-eau, une microémulsion, un gel aqueux, un gel anhydre, un sérum, une dispersion de vésicules, de microcapsules ou de microparticules.

5. Utilisation selon l'une quelconque des revendications précédentes, caractérisée en ce que la composition contient au moins un agent choisi parmi les agents antibactériens, antiparasitaires, antifongiques, anti-inflammatoires, antiprurigineux, anesthésiques, antiviraux, kératolytiques, antiradicaux libres, antiséborrhéiques, antipelliculaires, anti-acnéiques et/ou les agents modulant la différenciation et/ou la prolifération et/ou la pigmentation cutanée.

6. Utilisation selon l'une quelconque des revendications précédentes, caractérisée en ce que la composition est une composition topique pour prévenir et/ou lutter contre les irritations cutanées et/ou les dartres et/ou les érythèmes et/ou les sensations dysesthésiques et/ou les sensations d'échauffement et/ou les prurits de la peau et/ou des muqueuses.

7. Procédé de traitement cosmétique, caractérisé en ce que l'on applique sur la peau, sur le cuir chevelu et/ou sur les muqueuses une composition contenant au moins un composé choisi parmi les antagonistes de bradykinine dans un milieu cosmétiquement acceptable.

8. Procédé selon la revendication 7, caractérisé en ce que le composé est utilisé en une quantité allant de 0,000001 à 5 % en poids par rapport au poids total de la composition.

9. Procédé selon la revendication 7 ou 8, caractérisé en ce que le composé est utilisé en une quantité allant de 0,0001 à 0,1 % en poids par rapport au poids total de la composition.

10. Composition contenant dans un milieu cosmétiquement, pharmaceutiquement ou dermatologiquement acceptable au moins un produit à effet secondaire irritant, caractérisée en ce qu'elle contient au moins un agent antagoniste de cet effet choisi parmi les antagonistes de bradykinine.

11. Composition selon la revendication 10, caractérisée en ce que le compose est utilisé en une quantité allant de 0,000001 à 5 % en poids par rapport au poids total de la composition.

12. Composition selon l'une quelconque des revendications 10 et 11, caractérisée en ce que le composé est utilisé en une quantité allant de 0,0001 à 0,1 % en poids par rapport au poids total de la composition.

13. Composition selon l'une quelconque des revendications 10 à 12, caractérisée en ce que le produit à effet secondaire irritant est choisi parmi les α-hydroxy-acides, les β-hydroxy-acides, les α-céto-acides, les β-céto-acides, les rétinoïdes, les anthralines, les anthranoïdes, les peroxydes, le minoxidil, les sels de lithium, les antimétabolites, la vitamine D et ses dérivés, les dépigmentants, les solvants, les parfums, les conservateurs, les tensioactifs, les solutions alcooliques.

14. Composition selon l'une quelconque des revendications 10 à 13, caractérisée en ce qu'elle contient en outre au moins un agent choisi parmi les agents antibactériens, antiparasitaires, antiviraux, antifongiques, anti-inflammatoires, antiprurigineux, anesthésiques, kératolytiques, antiradicaux libres, antiséborrhéiques, antipelliculaires, anti-acnéiques et/ou les agents modulant la différenciation et/ou la prolifération et/ou la pigmentation cutanée.

15. Composition selon l'une quelconque des revendications 10 à 14, caractérisée en ce qu'elle contient au moins un actif à effet secondaire irritant choisi parmi les alpha-hydroxyacides et les bêta-hydroxyacides.

16. Composition selon l'une quelconque des revendications 10 à 15, caractérisée en ce que le milieu cosmétiquement et/ou dermatologiquement acceptable est une solution aqueuse, huileuse ou hydroalcoolique, une émulsion eau-dans-huile, une émulsion huile-dans-eau, une microémulsion, un gel aqueux, un gel anhydre, un sérum, une dispersion de vésicules.

## Claims

1. Use of at least one compound chosen from bradykinin antagonists, in a composition containing a cosmetically, dermatologically or pharmaceutically acceptable medium, for treating sensitive skin.

2. Use according to Claim 1, characterized in that the compound is used in an amount ranging from 0.000001 to 5 % by weight relative to the total weight of the composition.

3. Use according to either of the preceding claims, characterized in that the compound is used in an amount ranging from 0.0001 to 0.1 % by weight relative to the total weight of the composition.

4. Use according to any one of the preceding claims, characterized in that the cosmetically, pharmaceutically or dermatologically acceptable medium is an aqueous, oily or aqueous-alcoholic solution, a water-in-oil emulsion, an oil-in-water emulsion, a microemulsion, an aqueous gel, an anhydrous gel, a serum or a dispersion of vesicles, microcapsules or microparticles.

5. Use according to any one of the preceding claims, characterized in that the composition contains at least one agent chosen from antibacterial agents, antiparasitic agents, antifungal agents, anti-inflammatory agents, anti-pruriginous agents, anaesthetics, antiviral agents, keratolytic agents, anti-free-radical agents, antiseborrhoeic agents, antidandruff agents, anti-acne agents and/or agents which modify skin differentiation and/or proliferation and/or pigmentation.

6. Use according to any one of the preceding claims, characterized in that the composition is a topical composition, in order to prevent and/or combat skin irritations and/or dartres and/or erythema and/or dysaesthesic sensations and/or sensations of heating and/or pruritus of the skin and/or of the mucous membranes.

7. Cosmetic treatment process, characterized in that a composition containing at least one compound chosen from bradykinin antagonists in a cosmetically acceptable medium is applied to the skin, to the scalp and/or to the mucous membranes.

8. Process according to Claim 7, characterized in that the compound is used in an amount ranging from 0.000001 to 5 % by weight relative to the total weight of the composition.

9. Process according to Claim 7 or 8, characterized in that the compound is used in an amount ranging from 0.0001 to 0.1 % by weight relative to the total weight of the composition.

10. Composition containing, in a cosmetically, pharmaceutically or dermatologically acceptable medium, at least one product with an irritant side effect, characterized in that it contains at least one agent which is an antagonist of this effect, chosen from bradykinin antagonists.

11. Composition according to Claim 10, characterized in that the compound is used in an amount ranging from 0.000001 to 5 % by weight relative to the total weight of the composition.

12. Composition according to either of Claims 10 and 11, characterized in that the compound is used in an amount ranging from 0.0001 to 0.1 % by weight relative to the total weight of the composition.

13. Composition according to any one of Claims 10 to 12, characterized in that the product with an irritant side effect is chosen from α-hydroxy acids, β-hydroxy acids, α-keto acids, β-keto acids, retinoids, anthralins, anthranoids, peroxides, minoxidil, lithium salts, antimetabolites, vitamin D and derivatives thereof, depigmenting agents, solvents, fragrances, preserving agents, surfactants and alcoholic solutions.

14. Composition according to any one of Claims 10 to 13, characterized in that it also contains at least one agent chosen from antibacterial agents, antiparasitic agents, antiviral agents, antifungal agents, anti-inflammatory agents, anti-pruriginous agents, anaesthetics, keratolytic agents, anti-free-radical agents, antiseborrhoeic agents, antidandruff agents, anti-acne agents and/or agents which modify skin differentiation and/or proliferation and/or pigmentation.

15. Composition according to any one of Claims 10 to 14, characterized in that it contains at least one active agent with an irritant side effect, chosen from alpha-hydroxy acids and beta-hydroxy acids.

16. Composition according to any one of Claims 10 to 15, characterized in that the cosmetically and/or dermatologically acceptable medium is an aqueous, oily or aqueous-alcoholic solution, a water-in-oil emulsion, an oil-in-water emulsion, a microemulsion, an aqueous gel, an anhydrous gel, a serum or a dispersion of vesicles.

## Patentansprüche

1. Verwendung mindestens einer Verbindung, die unter den Bradykinin-Antagonisten ausgewählt ist, in einer Zusammensetzung, die ein kosmetisch, dermatologisch oder pharmazeutisch akzeptables Medium enthält, zur Behandlung von empfindlicher Haut.

2. Verwendung nach Anspruch 1, dadurch gekennzeichnet, daß die Verbindung in einem Mengenanteil von 0,000001 bis 5 Gew.-%, bezogen auf das Gesamtgewicht der Zusammensetzung, verwendet wird.

3. Verwendung nach einem der vorhergehenden Ansprüche, dadurch gekennzeichnet, daß die Verbindung in einem Mengenanteil von 0,0001 bis 0,1 Gew.-%, bezogen auf das Gesamtgewicht der Zusammensetzung, verwendet wird.

4. Verwendung nach einem der vorhergehenden Ansprüche, dadurch gekennzeichnet, daß das kosmetisch, pharmazeutisch oder dermatologisch akzeptable Medium eine wäßrige, ölige oder wäßrig-alkoholische Lösung, eine Wasser-in-Öl-Emulsion, eine Öl-in-Wasser-Emulsion, eine Mikroemulsion, ein wäßriges Gel, ein wasserfreies Gel, ein Serum, eine Vesikeldispersion, eine Mikrokapseldispersion oder eine Mikropartikeldispersion ist.

5. Verwendung nach einem der vorhergehenden Ansprüche, dadurch gekennzeichnet, daß die Zusammensetzung mindestens einen Wirkstoff enthält, der unter den antibakteriellen Mitteln, Mitteln gegen Parasiten, Mitteln gegen Pilze, Antiphlogistika, Mitteln gegen Juckreiz, Anästhetika, Mitteln gegen Viren, Keratolytika, Mitteln gegen freie Radikale, Mitteln gegen Seborrhoe, Mitteln gegen Schuppen, Mitteln gegen Anne und/oder Mitteln, die die Differenzierung und/oder Proliferation und/oder Pigmentierung der Haut modulieren, ausgewählt ist.

6. Verwendung nach einem der vorhergehenden Ansprüche, dadurch gekennzeichnet, daß die Zusammensetzung eine topische Zusammensetzung zur Vorbeugung und/oder Bekämpfung von Hautreizungen und/oder Flechten und/oder Erythemen und/oder unangenehme Empfindungen und/oder Hitzeempfindungen und/oder Juckreiz der Haut und/oder der Schleimhäute ist.

7. Verfahren zur kosmetischen Behandlung, dadurch gekennzeichnet, daß auf die Haut, die Kopfhaut und/oder die Schleimhäute eine Zusammensetzung aufgetragen wird, die in einem kosmetisch akzeptablen Medium mindestens eine Verbindung enthält, die unter den Bradykinin-Antagonisten ausgewählt ist.

8. Verfahren nach Anspruch 7, dadurch gekennzeichnet, daß die Verbindung einen Mengenanteil von 0,000001 bis 5 Gew.-%, bezogen auf das Gesamtgewicht der Zusammensetzung, verwendet wird.

9. Verfahren nach Anspruch 7 oder 8, dadurch gekennzeichnet, daß die Verbindung in einem Mengenanteil von 0,0001 bis 0,1 Gew.-%, bezogen auf das Gesamtgewicht der Zusammensetzung, verwendet wird.

10. Zusammensetzung, die in einem kosmetisch, pharmazeutisch oder dermatologisch akzeptablen Medium mindestens ein Produkt mit reizender Nebenwirkung enthält, dadurch gekennzeichnet, daß sie mindestens einen Antagonisten dieses Effekts enthält, der unter den Bradykinin-Antagonisten ausgewählt ist.

11. Zusammensetzung nach Anspruch 10, dadurch gekennzeichnet, daß die Verbindung in einem Mengenanteil von 0,000001 bis 5 Gew.-%, bezogen auf das Gesamtgewicht der Zusammensetzung, verwendet wird.

12. Zusammensetzung nach einem der Ansprüche 10 und 11, dadurch gekennzeichnet, daß die Verbindung in einem Mengenanteil von 0,0001 bis 0,1 Gew.-%, bezogen auf das Gesamtgewicht der Zusammensetzung, verwendet wird.

13. Zusammensetzung nach einem der Ansprüche 10 bis 12, dadurch gekennzeichnet, daß das Produkt mit reizender Nebenwirkung unter den α-Hydroxysäuren, β-Hydroxysäuren, α-Ketosäuren, β-Ketosäuren, Retinoiden, Anthralinen, Anthranoiden, Peroxiden, Minoxidil, Lithiumsalzen, Antimetaboliten, Vitamin D und seinen Derivaten, Depigmentierungsmitteln, Lösungsmitteln, Parfums, Konservierungsmitteln, grenzflächenaktiven Stoffen und alkoholischen Lösungen ausgewählt ist.

14. Zusammensetzung nach einem der Ansprüche 10 bis 13, dadurch gekennzeichnet, daß sie mindestens ein Mittel enthält, das unter den antibakteriellen Mitteln, Mitteln gegen Parasiten, Mitteln gegen Viren, Mitteln gegen Pilze, Antiphlogistika, Mitteln gegen Juckreiz, Anästhetika, Keratolytika, Mitteln gegen freie Radikale, Mitteln gegen Seborrhoe, Mitteln gegen Schuppen, Mitteln gegen Akne und/oder Mitteln, die die Differenzierung und/oder Proliferation und/oder Pigmentierung der Haut modulieren, ausgewählt ist.

15. Zusammensetzung nach einem der Ansprüche 10 bis 14, dadurch gekennzeichnet, daß sie mindestens einen Wirkstoff mit reizender Nebenwirkung enthält, der unter den α-Hydroxysäuren und β-Hydroxysäuren ausgewählt ist.

16. Zusammensetzung nach einem der Ansprüche 10 bis 15, dadurch gekennzeichnet, daß kosmetisch und/oder dermatologisch akzeptable Medium eine wäßrige, ölige oder wäßrigalkoholische Lösung, eine Wasser-in-Öl-Emulsion, eine Öl-in-Wasser-Emulsion, eine Mikroemulsion, ein wäßriges Gel, ein wasserfreies Gel, ein Serum oder eine Vesikeldispersion ist.
